# FASCICULE DE BREVET EUROPEEN

(11) **EP 3 292 405 B1**
(45) Date de publication et mention de la délivrance du brevet: **14.09.2022**
(21) Numéro de dépôt: 16720839.6
(22) Date de dépôt: 04.05.2016
(51) Int. Cl.: G01N 33/28

(54) **PROCEDE DE SUIVI EN CONTINU DE L'ETAT D'AVANCEMENT D'OXYDATION D'UN CARBURANT**
VERFAHREN ZUR KONTINUIERLICHEN ÜBERWACHUNG DES FORTSCHRITTS DER OXIDATION EINES BRENNSTOFFS
METHOD FOR CONTINUOUSLY MONITORING THE PROGRESS OF OXIDATION OF A FUEL

(30) Priorité: 05.05.2015 FR 1554011
(43) Date de publication de la demande: 14.03.2018
(73) Titulaire: IFP Energies nouvelles, 92500 Rueil-Malmaison (FR)
(72) Inventeur: ALVES FORTUNATO, Maira, 78420 Carrières-sur-Seine (FR); BEN AMARA, Arij, 78230 Le Pecq (FR); JEULAND, Nicolas, 92160 Antony (FR); STARCK, Laurie, 92500 Rueil Malmaison (FR)
(74) Mandataire: IFP Energies nouvelles
(86) Numéro de dépôt international: PCT/EP2016/060101
(87) Numéro de publication internationale: WO 2016/177838

(56) Documents cités:
- WO-A1-2008/080108
- JP-A- 2008 281 486
- US-A1- 2007 187 617
- DATABASE WPI Week 201372 Thomson Scientific, London, GB; AN 2013-R69140 XP002754855, & WO 2013/154175 A1 (NISSAN MOTOR CO LTD) 17 octobre 2013 (2013-10-17)
- MIKELIS SVILANS ET AL: "Spectroscopic Monitoring of Biodiesel Aging", RIGAS TEHNISKAS UNIVERSITATES ZINATNISKIE RAKSTI. SERIJA 1: MATERIALZINATNE UN LIETISKA KIMIJA, vol. 28, no. 28, 18 octobre 2013 (2013-10-18), page 85, XP055253987, Riga ISSN: 1407-7353, DOI: 10.7250/msac.2013.012

## Description

### DOMAINE DE L'INVENTION

L'évolution du contexte environnemental, économique et réglementaire a favorisé l'émergence des biocarburants et carburants alternatifs dans les distillats moyens comme les kérosènes et les diesels.

Ces évolutions concernent aussi bien la formulation des carburants, la logistique et les applications associées. Elles ont néanmoins été accompagnées par des problèmes de dégradation plus fréquents des carburants. Les carburants alternatifs à base d'esters méthyliques d'acide gras (EMAG) utilisés comme biocarburant apportent notamment des composants moins stables qui facilitent les phénomènes d'autoxydation et la formation de dépôt dans les circuits de carburants.

La formation de ces dépôts et vernis provenant de l'autoxydation du carburant, est critique pour les industries du transport aéronautique et terrestre. Ces vernis peuvent en effet entraîner des défaillances importantes des moteurs et turbines et donc être critiques pour la sécurité des utilisateurs. Il est donc fondamental de pouvoir déterminer de façon précise l'état d'oxydation d'un carburant, qu'il soit d'origine fossile ou issu de la biomasse, afin d'être en mesure d'éviter l'utilisation de carburants dont l'état d'oxydation favoriserait ces phénomènes.

### ART ANTÉRIEUR

La demande de brevet WO 14/085009 divulgue un modèle permettant de préparer un carburant de type kérosène dont le point de rupture thermique est prévisible en fonction de sa composition initiale en terme de composés soufrés, de composés nitrés, de composés aromatiques et de densité. La méthode décrite est basée sur une caractérisation de type spectroscopie de masse à transformée de Fourrier par résonnance cyclotronique des ions (FTICR-MS), entre autres, des kérosènes aéronautiques afin de déterminer leur stabilité thermique.

La demande de brevet US 2008/167823 décrit une méthode de mesure de la variation de la concentration des esters ou de la présence d'acides gras, de glycérides ou de méthanol au travers de l'utilisation de la spectroscopie d'impédance. La méthode décrite permet de mesurer la concentration en ester méthylique d'acide gras (EMAG) dans un carburant de type diesel ou kérosène ainsi que de mesurer l'indice d'acide (nombre d'acide totale : TAN) considéré comme un indicateur de stabilité et de dégradation des carburants.

La demande de brevet JP 2008 281486 décrit une autre méthode de détermination de l'oxidation d'un carburant par la mesure du nombre d'acide total (TAN).

Le brevet US 5 163 982 décrit une méthode de détection des espèces polaires présentes en se basant sur la chromatographie en phase gaz (CPG) en phase inverse qui permet de déterminer et classer par méthode comparative la stabilité de carburants.

La demande de brevet WO 09/080049 propose une méthode de mesure, (proche infrarouge, fluorescence) en ligne ou hors ligne, et de corrélation par méthode chimio-métriques avec des caractéristiques de carburant dont le TAN pour prédire les propriétés et les qualités d'un carburants lors de sa préparation.

Le brevet US 5 475 612 et la demande de brevet WO 94/17391 proposent une méthode de prédiction des propriétés d'un carburant grâce à une corrélation entre le spectre infrarouge dudit carburant et ses propriétés physico-chimique, une application possible étant la prédiction des propriétés de type indice d'octane « recherche » (RON) ou indice de cétane d'un carburant.

La spectrométrie en générale et en particulier la spectroscopie infrarouge (IR) permet de corréler les spectres obtenus aux propriétés d'un carburant. Les propriétés couramment déterminées ainsi sont, la densité, la viscosité ou encore l'indice d'octane. La stabilité à l'oxydation et l'état d'avancement d'oxydation d'un carburant ne sont pas déterminés par ces techniques classiques d'analyses chimique.

Par exemple, la stabilité thermique et la stabilité à l'oxydation d'un carburant sont, dans le domaine aéronautique, généralement déterminées par l'utilisation de la méthode dite testeur d'oxydation thermique du kérosène (jet fuel thermal oxydation tester : JFTOT) qui donne un résultat de type « réussite ou échec ».

Actuellement, pour la plupart, les méthodes de caractérisation de la stabilité à l'oxydation des carburants sont basées sur une oxydation forcée du produit en température avec un suivi de l'oxydation par la consommation d'oxygène ou la caractérisation des produits formés ou sur l'évolution d'une propriété (indice d'acide, conductivité...). Néanmoins, ces méthodes ne permettent pas d'avoir une corrélation fiable entre le potentiel de formation de vernis et l'avancement réel de l'état d'oxydation du carburant à un instant donné. Il est ainsi souvent nécessaire de coupler plusieurs méthodes afin d'avoir une vision exhaustive.

Par ailleurs, plusieurs techniques d'analyse telles que l'infrarouge à transformée de fourrier (FTIR), la chromatographie en phase gaz (CPG) ou encore la spectroscopie d'impédance ont été proposées pour déterminer la stabilité d'un carburant. Cependant ces techniques permettent seulement la mesure à l'état initial ou à un instant t. A notre connaissance seule la demande de brevet WO 09/080049 propose une méthode de suivi en continu pouvant être mise en œuvre au sein d'un procédé de production de carburant.

Il serait donc très avantageux de développer une méthode permettant de suivre la dégradation des carburants en continu et d'alerter l'utilisateur en cas de forte dégradation, en se basant sur l'évolution de la composition chimique de ces carburants en cours d'utilisation et en fonction de la température.

La demanderesse a pu développer un tel procédé innovant permettant de suivre en continu l'évolution d'un carburant en vue de pouvoir déterminer à chaque instant si la composition et la qualité dudit carburant est suffisamment préservée pour permettre sa consommation.

### OBJET DE L'INVENTION

En particulier, la présente invention concerne un procédé de suivi en continu de l'état d'avancement d'oxydation d'un carburant selon la revendication 1.

Ainsi, le procédé selon l'invention présente une approche complémentaire et différente d'appréciation de la qualité d'un carburant, par rapport à l'état de la technique actuelle, grâce à la détermination de l'état d'oxydation dudit carburant.

Un avantage de la présente invention est de permettre de suivre la dégradation des carburants en continu et d'alerter l'utilisateur en cas de forte dégradation, en se basant sur l'évolution de la composition chimique de ces carburants en cours d'utilisation et en fonction de la température.

Un autre avantage de la présente invention est de fournir un procédé innovant de détermination de l'état de dégradation d'un carburant, basé sur le suivi en continu de diverses molécules, ou familles de molécules représentatives d'un état d'avancement de l'oxydation en phase liquide et en phase gaz.

Par ailleurs, le procédé selon la présente invention permet avantageusement la mesure de l'état d'avancement d'oxydation d'un carburant en phase logistique ou en phase de stockage à bord.

### BREVE DESCRIPTION DES FIGURES

La figure 1 est une superposition de spectres infra-rouge montrant l'évolution de l'état du carburant modèle frais suite à différentes périodes d'oxydations.
La figure 2 est un chromatogramme en phase gazeuse montrant l'état du carburant modèle frais (mélange n-paraffines et esters insaturés) à 0 IP lorsque le carburant modèle frais n'a pas encore subi d'oxydation.
La figure 3 est un chromatogramme en phase gazeuse montrant l'état du carburant modèle frais suite à une période d'oxydation.

### DESCRIPTION DÉTAILLÉE DE L'INVENTION

Dans toute la suite du texte, on définit les états d'avancement d'oxydation comme : phase initiale, phase intermédiaire et phase avancée. Les réactions d'oxydation des carburants présentent des cinétiques chimiques complexes avec de multiples intermédiaires réactionnels. Ces intermédiaires réactionnels sont des indicateurs de l'état d'avancement desdites réactions d'oxydation.

L'état d'avancement d'une réaction d'oxydation est donc directement corrélé à la quantité d'intermédiaires réactionnels, de co-produits et à la quantité de produits finaux, lesdits produits résultants d'au moins une des réactions d'oxydation avec un ou plusieurs composants du carburant puisque la composition chimique du carburant varie en fonction de l'état d'oxydation dans lequel il se trouve. Ainsi, il est possible de déterminer si un carburant se trouve en phase initiale d'oxydation, en phase intermédiaire d'oxydation ou en phase avancée d'oxydation par la détermination des teneurs desdits intermédiaires réactionnels, desdits coproduits et/ou desdits produits finaux.

La présente invention est donc relative à un procédé de suivi en continu de l'état d'avancement d'oxydation d'un carburant comprenant au moins les étapes suivantes :
a) Détermination d'au moins un indicateur d'avancement de réaction d'oxydation à suivre,
b) Mesure de la teneur dudit indicateur d'avancement de réaction d'oxydation dans ledit carburant,
c) Classification de l'état d'avancement d'oxydation dudit carburant,
d) Détermination des mesures à mettre en œuvre en fonction de ladite classification,
caractérisé en ce que le dit indicateur d'avancement est au moins un produit intermédiaire ou final ou au moins un coproduit d'au moins une des réactions d'oxydation avec un ou plusieurs composants du carburant,
et en ce que la classification est effectuée par comparaison entre lesdites teneurs dudit indicateur d'avancement mesurées et les teneurs en ledit indicateur d'avancement présents dans ledit carburant après une période d'oxydation forcée, mesurée selon la méthode EN15751 (ou EN14112, ASTM D6751) permettant de déterminer la période d'induction (IP) dudit carburant, ledit état d'avancement d'oxydation étant alors :
- Initiale si lesdites teneurs mesurées correspondent à une période d'oxydation (P1) strictement inférieure à 0,5 IP (0 IP ≤ P1 < 0,5 IP),
- Intermédiaire si lesdites teneurs mesurées correspondent à une période d'oxydation (P2) supérieure ou égale à 0,5 IP et strictement inférieure à 1 IP (0,5 IP ≤ P2 < IP),
- Avancée si lesdites teneurs mesurées correspondent à une période d'oxydation (P3) supérieure ou égale à 1 IP (1 IP ≤ P3).

Le procédé selon la présente invention est avantageusement applicable à tout carburant d'origine fossile ou issu de la biomasse.

Dans un mode de réalisation préféré, le carburant est choisi parmi le kérosène, le gazole et l'essence.

Le procédé selon la présente invention permet avantageusement la mesure de l'état d'avancement d'oxydation d'un carburant en phase logistique ou en phase de stockage à bord.

On entend par phase de logistique l'ensemble des phases au cours desquelles un carburant n'est pas dans un réservoir de véhicule à moteur.

On entend par phase de stockage à bord l'ensemble des phases au cours desquelles un carburant est dans un réservoir de véhicule à moteur, que le moteur soit en fonctionnement ou non.

Conformément à l'invention, le procédé comprend une étape a) de détermination d'au moins un indicateur d'avancement de réaction d'oxydation à suivre.

Selon l'invention, ledit indicateur d'avancement est un produit intermédiaire, un produit final ou un coproduit d'au moins une des réactions d'oxydation avec un ou plusieurs composants du carburant.

L'oxydation d'hydrocarbures conduit irrémédiablement à la formation de molécules porteuses d'au moins une fonction oxygénée. En fonction de l'état d'avancement d'oxydation, les molécules porteront des fonctions comportant plus ou moins d'atomes d'oxygènes. Par exemple une fonction de type acide carboxylique qui comporte deux atomes d'oxygènes est issue de l'oxydation d'une fonction aldéhyde qui comporte un seul atome d'oxygène. Le suivi de la formation et de l'évolution des molécules porteuses d'au moins une fonction oxygénée au sein d'un carburant permet donc de déterminer l'état d'oxydation dudit carburant.

L'indicateur d'avancement est avantageusement une ou plusieurs molécules appartenant aux familles porteuses d'au moins une fonction oxygénée ou encore la distribution de poids moléculaire d'une ou de plusieurs molécules polymérisées issues de monomères appartenant à ces familles.

Dans un premier mode de réalisation, l'indicateur d'avancement est de préférence une ou plusieurs molécules appartenant aux familles choisies parmi les aldéhydes, les alcools, les cétones, les cétoacides, les époxydes et les acides carboxyliques.

Les molécules composant ledit carburant avant oxydation appartiennent de manière préférée à la famille des alcanes linéaires ou ramifiés, alcènes linéaires ou ramifiés ou la famille des aromatiques

De manière préférée, ledit indicateur d'avancement est une molécule spécifique appartenant aux familles définies ci-dessus.

Dans le cas où l'indicateur d'avancement est une molécule appartenant à la famille des aldéhydes, l'indicateur d'avancement est choisi parmi le formaldéhyde et l'acétaldéhyde.

Dans le cas où l'indicateur d'avancement est une molécule appartenant à la famille des alcools, l'indicateur d'avancement est le méthanol.

Dans le cas où l'indicateur d'avancement est une molécule appartenant à la famille des époxydes, l'indicateur d'avancement est l'oxyde d'éthylène.

De manière alternative l'indicateur d'avancement est un ou plusieurs co-produit(s) issus des différentes réactions d'oxydation que ledit carburant peut subir.

Dans le cas où l'indicateur d'avancement est un ou plusieurs coproduits, ledit indicateur d'avancement est avantageusement une ou plusieurs molécules polymérisées issues d'un ou plusieurs monomères.

De manière préférée, lesdits monomères appartiennent aux familles des alcènes linéaires ou ramifiés, des aromatiques, des aldéhydes, des alcools, des cétones, des cétoacides, des époxydes et des acides carboxyliques.

Dans le cas où ledit monomère appartient à la famille des aldéhydes, le monomère est de préférence choisi parmi le formaldéhyde et l'acétaldéhyde.

Dans le cas où ledit monomère appartient à la famille des alcools, le monomère est de préférence le méthanol.

Dans le cas où ledit monomère appartient à la famille des époxydes, le monomère est de préférence l'oxyde d'éthylène.

Dans le cas ledit monomère appartient à la famille des peroxydes, le monomère est choisi de préférence parmi les peroxydes, alkoxydes d'espèces radicalaires (RO₂°, RO°, R°) dudit carburant.

Conformément à l'invention, le procédé comprend une étape b) de mesure de la teneur dudit indicateur d'avancement de réaction d'oxydation dans ledit carburant.

Dans un mode de réalisation, ladite mesure de la teneur d'au moins un indicateur d'avancement est effectuée par l'intermédiaire d'au moins une méthode d'analyse connue de l'homme du métier, telles que l'analyse par Ultra-Violet (UV), par Infra-Rouge (IR), par chromatographie en phase gazeuse (GC), par spectrométrie de masse (MS), par diffusion des rayon X aux petits angles (SAXS) ou encore par tonométrie.

Conformément à l'invention, le procédé comprend une étape c) de classification de l'état d'avancement d'oxydation dudit carburant.

Selon l'invention, la classification est effectuée par comparaison entre lesdites teneurs dudit indicateur d'avancement mesurées et les teneurs en ledit indicateur d'avancement présents dans ledit carburant après une période d'oxydation forcée, mesurée selon la méthode EN 15751 (EN14112, ASTM D 6751) permettant de déterminer la période d'induction (IP) dudit carburant.

La période d'induction, ou IP est le temps nécessaire au carburant pour atteindre un état d'oxydation définit. Les carburants, selon leur destination et leur territoire d'utilisation doivent présenter un IP supérieur à une certaine durée pour respecter les spécifications minimales.

Dans la méthode standard EN 15751, un échantillon de 7.5 g de carburant est soumis à un bullage d'air (10 l / h), à une température spécifiée de (383K). L'air qui ressort de l'échantillon est piégé dans un flacon contenant de l'eau distillée, où la conductivité est mesurée. L'augmentation de la conductivité de la cellule indique une accumulation des acides volatiles dans l'eau, due à l'oxydation de l'échantillon. L'IP est caractérisée par un changement de pente de la conductimétrie de l'eau distillée.

Selon l'invention, ledit état d'avancement d'oxydation dudit carburant est exprimé en fonction de l'IP et est qualifié de :
- Initiale si lesdites teneurs mesurées correspondent à une période d'oxydation (P1) strictement inférieure à 0,5 IP (0 IP ≤ P1< 0,5 IP),
- Intermédiaire si lesdites teneurs mesurées correspondent à une période d'oxydation (P2) supérieure ou égale à 0,5 IP et strictement inférieure à 1 IP (0,5 IP ≤ P2 < IP),
- Avancée si lesdites teneurs mesurées correspondent à une période d'oxydation (P3) supérieure ou égale à 1 IP (1 IP ≤ P3).

La période d'induction représente donc les bornes définissant les trois états d'oxydation de la classification selon l'invention. Par exemple, si la teneur mesurée du ou des indicateurs d'avancement montre que la composition dudit carburant correspond à une période d'oxydation strictement inférieure à 0,5 IP, alors le carburant sera classé comme étant en phase initiale d'oxydation.

Conformément à l'invention, le procédé comprend une étape d) de détermination des actions à mener en fonction de ladite classification. L'utilisateur est informé de l'état de dégradation du carburant et le cas échéant des actions préventives ou curatives à mener.

Lorsque le carburant est en phase initiale d'oxydation, lesdites actions à mener sont avantageusement des actions correctives comme l'ajout d'additif antioxydant, ou de manière préférée, préventives comme l'élimination de sources pro-oxydantes.

Ledit additif antioxydant est avantageusement choisi parmi l'hydroxytoluène butylé (BHT), le 2,4-di-tert-butyl-phénol (2,4-DTBP), le 2-tert-butyl-4-méthylphénol (TBMP) et la Triphénylphosphine (TPP).

Lesdites sources pro-oxydantes peuvent être par exemple une trop forte aération, une trop forte luminosité ou encore la présence d'une source de contamination, il convient alors de limiter l'exposition du carburant aux dites sources pro-oxydantes.

Lorsque le carburant est en phase intermédiaire d'oxydation, lesdites actions à mener sont avantageusement des actions correctives comme l'ajout de carburant frais pour améliorer la qualité du produit. Dans un mode de réalisation l'action à mener sera d'informer l'utilisateur qu'une utilisation rapide dudit carburant en phase intermédiaire d'oxydation est préférable.

Lorsque le carburant est en phase avancée d'oxydation, lesdites actions à mener sont entre autres d'avertir l'utilisateur du risque avéré de l'utilisation dudit carburant.

### EXEMPLES

### Exemple 1 : Elaboration de la classification selon l'invention.

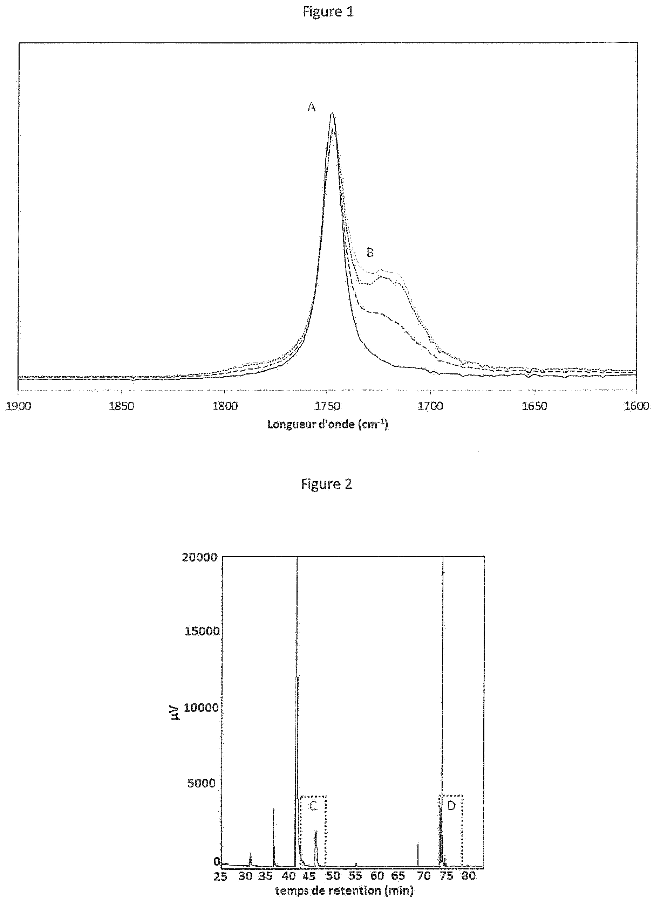

On prélève différents échantillons de 1 mL du carburant après différents temps d'oxydation. Les échantillons sont analysés avec un appareil FTIR (Fourier Transform Infrared Spectroscopy) BRUKER Vertex 70 sous balayage air sans H₂O/CO₂ avec résolution utilisé 2 cm⁻¹, détecteur DTGS, 32 scans par spectre et utilisation cuve en KBr. La Figure 1 montre l'évolution des spectres FTIR d'un carburant modèle subissant une oxydation. Le pic d'absorbance des oléfines est indiqué sur la figure par la lettre A et le pic d'absorbance des molécules oxygénées est indiqué sur la figure par la lettre B. La courbe en trait plein correspond à une période d'oxydation équivalente à 0 IP. La courbe en tirets correspond à une période d'oxydation de 0,5 IP. La courbe en pointillés épais correspond à une période d'oxydation de 1 IP. La courbe en pointillés fins correspond à une période d'oxydation de1,5 IP. Les résultats sont répertoriés dans le tableau 1.

**Tableau 1**

| | | | | |
|---|---|---|---|---|
| Période d'oxydation | 0 IP | 0,5 IP | 1 IP | 1,5 IP |
| Teneur en molécules oxygénées (hors oléfines oxygènes) (%m/m) | 0% | 1% | 3% | 4.3% |

Ainsi, dans l'exemple présent une teneur en molécules oxygénées strictement inférieur à 1% correspond à la période P1 soit un état d'avancement d'oxydation dit initiale selon l'invention, une teneur en molécules oxygénées supérieure ou égale à 1% et strictement inférieure 3% correspond à la période P2 soit un état d'avancement d'oxydation dit intermédiaire selon l'invention et une teneur en molécules oxygénées supérieure ou égale à 3% correspond à la période P3 soit un état d'avancement d'oxydation dit avancée selon l'invention.

### Exemple 2 : Utilisation de la spectroscopie de masse pour la mise en œuvre de l'invention.

On prélève un échantillon de 1 mL du carburant dont on veut connaitre l'état d'avancement d'oxydation. L'échantillon est analysé avec un appareil GC/FID, (colonne DB1 60m, 0,320 mm, 0,25 µm). Le chromatogramme de la figure 2 montre l'état du carburant modèle frais, la teneur en molécules oxygénées est de 0,0 à 0 IP, c'est-à-dire, il n'y a pas de produits d'oxydation présents dans le produit frais.

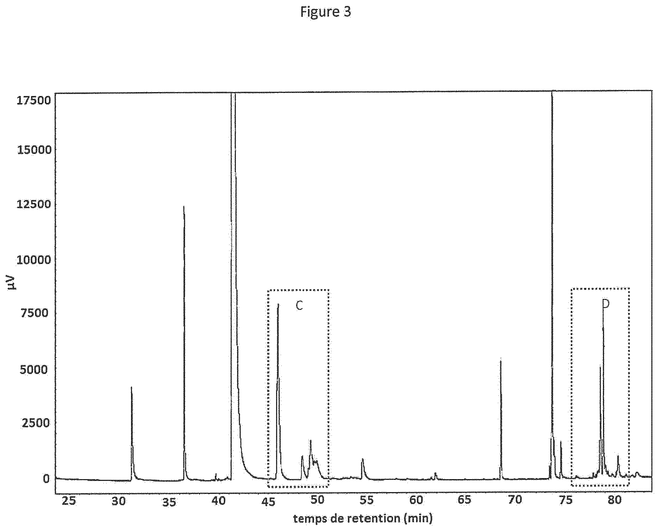

Le chromatogramme de la figure 3 montre l'état du carburant duquel a été prélevé l'échantillon. Les teneurs en cétones et époxydes sont mesurées, les résultats sont répertoriés dans le tableau 2.

Sur les chromatogrammes des figures 2 et 3, la zone des temps de rétention correspondants aux cétones est indiquée par la lettre C, la zone des temps de rétention corresponds aux époxydes est indiquée par la lettre D.

**Tableau 2**

| **Famille de molécule** | **Teneur (%m/m)** |
|---|---|
| Cétones | 3,4 |
| époxydes | 0,8 |
| Molécules oxygénées (cétones + époxydes) | 4.2 |

Les teneurs en molécules oxygénées correspondent à une période d'oxydation supérieure à 1 IP. Selon l'invention ce résultat correspond à une période d'oxydation P3. Le carburant de l'exemple 2 est donc classé dans la catégorie « état d'oxydation avancée ». L'action à mener est d'avertir l'utilisateur du risque avéré de l'utilisation du produit.

## Revendications

1. Procédé de suivi en continu de l'état d'avancement d'oxydation d'un carburant comprenant au moins les étapes suivantes :
a) Détermination d'au moins un indicateur d'avancement de réaction d'oxydation à suivre,
b) Mesure de la teneur dudit indicateur d'avancement de réaction d'oxydation dans ledit carburant,
c) Classification de l'état d'avancement d'oxydation dudit carburant,
d) Détermination des mesures à mettre en œuvre en fonction de ladite classification,
**caractérisé en ce que** le dit indicateur d'avancement est au moins un produit intermédiaire ou final ou au moins un coproduit d'au moins une des réactions d'oxydation avec un ou plusieurs composants du carburant, **en ce qu'**on détermine si un carburant se trouve en phase initiale d'oxydation, en phase intermédiaire d'oxydation ou en phase avancée d'oxydation par la détermination des teneurs desdits intermédiaires réactionnels, desdits coproduits et/ou desdits produits finaux, et **en ce que** ledit indicateur d'avancement est une ou plusieurs molécules appartenant aux familles choisies parmi les aldéhydes, les alcools, les cétones, les cétoacides, les époxydes et les acides carboxyliques ou **en ce que** ledit indicateur d'avancement est au moins un co-produit, ledit co-produits étant une ou plusieurs molécules polymérisées issues d'un ou plusieurs monomères appartenant aux familles des alcènes linéaires ou ramifiés, des aromatiques, des aldéhydes, des alcools, des cétones, des cétoacides, des époxydes et des acides carboxyliques.
et **en ce que** la classification est effectuée par comparaison entre lesdites teneurs dudit indicateur d'avancement mesurées et les teneurs en ledit indicateur d'avancement présents dans ledit carburant après une période d'oxydation forcée, mesurée selon la méthode EN 15751 permettant de déterminer la période d'induction (IP) dudit carburant, ledit état d'avancement d'oxydation étant alors :
• Initiale si lesdites teneurs mesurées correspondent à une période d'oxydation (P1) strictement inférieure à 0,5 IP (0 IP ≤ P1 < 0,5 IP),
• Intermédiaire si lesdites teneurs mesurées correspondent à une période d'oxydation (P2) supérieure ou égale à 0,5 IP et strictement inférieure à 1 IP (0,5 IP ≤ P2 < IP),
• Avancée si lesdites teneurs mesurées correspondent à une période d'oxydation (P3) supérieure ou égale à 1 IP (1 IP ≤ P3).

2. Procédé selon la revendication 1, **caractérisé en ce que** le carburant est choisi parmi le kérosène, le gazole et l'essence.

3. Procédé selon l'une des revendications précédentes, **caractérisé en ce que** l'indicateur d'avancement est une molécule spécifique choisie parmi les familles des aldéhydes, des alcools, des cétones, des cétoacides, des époxydes et des acides carboxyliques.

4. Procédé selon l'une des revendications précédentes, **caractérisé en ce que** l'indicateur d'avancement est une molécule spécifique choisie parmi le formaldéhyde et l'acétaldéhyde.

5. Procédé selon l'une des revendications précédentes, **caractérisé en ce que** l'indicateur d'avancement est l'oxyde d'éthylène.

6. Procédé selon la revendication 1, **caractérisé en ce que** ledit monomère appartient à la famille des aldéhydes et choisi parmi le formaldéhyde et l'acétaldéhyde.

7. Procédé selon la revendication 1, **caractérisé en ce que** ledit monomère appartient à la famille des époxydes.

8. Procédé selon l'une des revendications précédentes, **caractérisé en ce que** le carburant est en phase de stockage à bord.

9. Procédé selon l'une des revendications précédentes, **caractérisé en ce que** le carburant est en phase de logistique.

10. Procédé selon l'une des revendications précédentes, **caractérisé en ce que** lorsque ledit carburant est en phase initiale d'oxydation, les actions à mener sont des actions correctives comme l'ajout d'additif antioxydant, ou préventives comme l'élimination de sources pro-oxydantes.

11. Procédé selon l'une des revendications précédentes, **caractérisé en ce que** lorsque le carburant est en phase intermédiaire d'oxydation, les actions à mener sont des actions correctives comme l'ajout de carburant frais pour améliorer la qualité du produit.

12. Procédé selon l'une des revendications précédentes, **caractérisé en ce que** lorsque le carburant est en phase avancée d'oxydation, une action à mener est d'avertir l'utilisateur du risque avéré de l'utilisation dudit carburant.

## Patentansprüche

1. Verfahren zum kontinuierlichen Überwachen des Fortschrittsstaus der Oxidation eines Kraftstoffs, das mindestens die folgenden Schritte umfasst:
a) Bestimmung mindestens eines Indikators für den Fortschritt der zu überwachenden Oxidationsreaktion,
b) Messen des Gehalts des Indikators für den Fortschritt der Oxidationsreaktion im Kraftstoff,
c) Einstufung des Fortschrittsstaus der Oxidation des Kraftstoffs,
d) Festlegen von Maßnahmen, die je nach der Einstufung zu ergreifen sind,
**dadurch gekennzeichnet, dass** der Fortschrittsindikator mindestens ein Zwischen- oder Endprodukt oder mindestens ein Nebenprodukt mindestens einer der Oxidationsreaktionen mit einem oder mehreren Bestandteilen des Kraftstoffs ist, dadurch, dass man durch Bestimmung der Gehalte der Reaktionszwischenprodukte, der Nebenprodukte und/oder der Endprodukte bestimmt, ob sich ein Kraftstoff in der Anfangsphase der Oxidation, in einer intermediären Oxidationsphase oder in einer fortgeschrittenen Oxidationsphase befindet, und dadurch, dass es sich bei dem Fortschrittsindikator um ein oder mehrere Moleküle handelt, die zu den aus Aldehyden, Alkoholen, Ketonen, Ketosäuren, Epoxiden und Carbonsäuren ausgewählten Familien gehören, oder dadurch, dass der Fortschrittsindikator mindestens ein Nebenprodukt ist, wobei es sich bei den Nebenprodukten um ein oder mehrere polymerisierte Moleküle handelt, die von einem oder mehreren Monomeren herrühren, die zu den Familien der geraden oder verzweigten Alkene, der Aromaten, der Aldehyde, der Alkohole, der Ketone, der Ketosäuren, der Epoxide und der Carbonsäuren gehören,
und dadurch, dass die Einstufung durchgeführt wird durch Vergleich zwischen den gemessenen Gehalten des Fortschrittsindikators und den Gehalten an dem Fortschrittsindikator, die in dem Kraftstoff nach einem Zeitraum der beschleunigten Oxidation vorhanden sind, die gemäß dem Verfahren EN 15751 gemessen werden, was es gestattet, die Induktionsperiode (IP) des Kraftstoffs zu bestimmen, wobei der Fortschrittsstatus der Oxidation dann:
• anfänglich ist, wenn die gemessenen Gehalte einer Oxidationsperiode (P1) von strikt weniger als 0,5 IP (0 IP ≤ P1 < 0,5 IP) entsprechen,
• intermediär ist, wenn die gemessenen Gehalte einer Oxidationsperiode (P2) von mehr als oder gleich 0,5 IP und strikt weniger als 1 IP (0,5 IP ≤ P2 < IP) entsprechen,
• fortgeschritten ist, wenn die gemessenen Gehalte einer Oxidationsperiode (P3) von mehr als oder gleich 1 IP (1 IP ≤ P3) entsprechen.

2. Verfahren nach Anspruch 1, **dadurch gekennzeichnet, dass** der Kraftstoff aus Kerosin, Gasöl und Benzin ausgewählt ist.

3. Verfahren nach einem der vorhergehenden Ansprüche, **dadurch gekennzeichnet, dass** der Fortschrittsindikator ein spezifisches, aus den Familien der Aldehyde, der Alkohole, der Ketone, der Ketosäuren, der Epoxide und der Carbonsäuren ausgewähltes Molekül ist.

4. Verfahren nach einem der vorhergehenden Ansprüche, **dadurch gekennzeichnet, dass** der Fortschrittsindikator ein spezifisches Molekül ist, das aus Formaldehyd oder Acetaldehyd ausgewählt ist.

5. Verfahren nach einem der vorhergehenden Ansprüche, **dadurch gekennzeichnet, dass** der Fortschrittsindikator Ethylenoxid ist.

6. Verfahren nach Anspruch 1, **dadurch gekennzeichnet, dass** das Monomer zur Familie der Aldehyde gehört und aus Formaldehyd und Acetaldehyd ausgewählt ist.

7. Verfahren nach Anspruch 1, **dadurch gekennzeichnet, dass** das Monomer zur Familie der Epoxide gehört.

8. Verfahren nach einem der vorhergehenden Ansprüche, **dadurch gekennzeichnet, dass** sich der Kraftstoff in der Phase der Lagerung an Bord befindet.

9. Verfahren nach einem der vorhergehenden Ansprüche, **dadurch gekennzeichnet, dass** sich der Kraftstoff in der Logistikphase befindet.

10. Verfahren nach einem der vorhergehenden Ansprüche, **dadurch gekennzeichnet, dass**, wenn sich der Kraftstoff in der Anfangsphase der Oxidation befindet, die zu ergreifenden Maßnahmen korrigierende Maßnahmen, wie Zugabe eines antioxidativen Zusatzstoffs, oder präventive Maßnahmen, wie Beseitigung oxidationsfördernder Quellen, sind.

11. Verfahren nach einem der vorhergehenden Ansprüche, **dadurch gekennzeichnet, dass**, wenn sich der Kraftstoff in der intermediären Oxidationsphase befindet, die zu ergreifenden Maßnahmen korrigierende Maßnahmen, wie Zugabe von frischem Kraftstoff zur Verbesserung der Qualität des Produkts, sind.

12. Verfahren nach einem der vorhergehenden Ansprüche, **dadurch gekennzeichnet, dass**, wenn sich der Kraftstoff in der fortgeschrittenen Oxidationsphase befindet, eine zu ergreifende Maßnahme ist, den Benutzer vor der echten Gefahr der Verwendung des Kraftstoffs zu warnen.

## Claims

1. Process for the continuous monitoring of the state of advancement of the oxidation of a fuel, comprising at least the following steps:
a) determining at least one indicator of advancement of the oxidation reaction to be monitored,
b) measuring the content of said indicator of advancement of the oxidation reaction in said fuel,
c) classifying the state of advancement of the oxidation of said fuel,
d) determining the measures to be implemented as a function of said classification,
**characterized in that** said indicator of advancement is at least one intermediate or end product, or at least one co-product, of at least one of the oxidation reactions with one or more constituents of the fuel, **in that** the process determines whether a fuel is in an initial stage of oxidation, in an intermediate stage of oxidation or in an advanced stage of oxidation by determining the contents of said reaction intermediates, said co-products and/or said end products, and **in that** said indicator of advancement is one or more molecules belonging to families selected from aldehydes, alcohols, ketones, ketoacids, epoxides and carboxylic acids, or **in that** said indicator of advancement is at least one co-product, said co-products being one or more polymerized molecules derived from one or more monomers belonging to the families of linear or branched alkenes, of aromatics, of aldehydes, of alcohols, of ketones, of ketoacids, of epoxides and of carboxylic acids,
and **in that** the classification is accomplished by comparing said measured contents of said indicator of advancement with the contents of said indicator of advancement present in said fuel after a period of forced oxidation, measured in accordance with EN 15751 which permits the determination of the induction period (IP) of said fuel, said state of advancement of oxidation then being:
• initial, if said measured contents correspond to a period of oxidation (P1) of strictly less than 0.5 IP (0 IP ≤ P1 < 0.5 IP),
• intermediate, if said measured contents correspond to a period of oxidation (P2) of greater than or equal to 0.5 IP and strictly less than 1 IP (0.5 IP ≤ P2 < IP),
• advanced, if said measured contents correspond to a period of oxidation (P3) of greater than or equal to 1 IP (1 IP ≤ P3).

2. Process according to Claim 1, **characterized in that** the fuel is selected from kerosene, diesel and gasoline.

3. Process according to either of the preceding claims, **characterized in that** the indicator of advancement is a specific molecule selected from the families of aldehydes, of alcohols, of ketones, of ketoacids, of epoxides and of carboxylic acids.

4. Process according to one of the preceding claims, **characterized in that** the indicator of advancement is a specific molecule selected from formaldehyde and acetaldehyde.

5. Process according to one of the preceding claims, **characterized in that** the indicator of advancement is ethylene oxide.

6. Process according to Claim 1, **characterized in that** said monomer belongs to the family of aldehydes and selected from formaldehyde and acetaldehyde.

7. Process according to Claim 1, **characterized in that** said monomer belongs to the family of epoxides.

8. Process according to one of the preceding claims, **characterized in that** the fuel is in the on-board storage stage.

9. Process according to one of the preceding claims, **characterized in that** the fuel is in the logistics stage.

10. Process according to one of the preceding claims, **characterized in that**, when said fuel is in an initial stage of oxidation, the actions to be taken are corrective actions such as the addition of antioxidant additive, or preventive actions such as elimination of pro-oxidant sources.

11. Process according to one of the preceding claims, **characterized in that**, when the fuel is in an intermediate stage of oxidation, the actions to be taken are corrective actions such as the addition of fresh fuel to improve the quality of the product.

12. Process according to one of the preceding claims, **characterized in that**, when the fuel is in an advanced stage of oxidation, one action to be taken is to warn the user of the demonstrated risk of using said fuel.
